# EUROPEAN PATENT APPLICATION

(11) **EP 2 556 825 A1**
(43) Date of publication of application: **13.02.2013**
(21) Application number: 12191988.0
(22) Date of filing: 24.07.2003
(51) Int. Cl.: A61K 9/20, A61K 9/48, A61J 3/07

(54) **Multicomponent pharmaceutical dosage form**

(30) Priority: 25.07.2002 GB 0217336; 03.02.2003 GB 0302435
(62) Divisional of application: 03740843.2
(71) Applicant: Capsugel Belgium NV, 2880 Bornem (BE)
(72) Inventor: Bonney, Stanley, George, Ware, Essex SG12 0DP (GB); Brown, Adrian, Harlow, Essex CM19 5AW (GB); Davies, Michael Birsha, Ware, Hertfordshire SG12 0DP (GB); Matthews, Wayne, M, Harlow, Essex CM19 5AW (GB); McAllister, Stephen, Mark, Harlow, Essex CM19 5AW (GB); Rand, Paul, Kenneth, Ware, Essex SG12 0DP (GB); Wilson, Alan, Anthony, Ware, Hertfordshire SG12 0DP (GB); Margetson, Daniel, N, Harlow, Essex CM19 5AW (GB)
(74) Representative: Krishnan, Sri

(57) **Abstract**

The present invention relates to a multi-component pharmaceutical dosage form for retaining a drug substance to provide a controlled drug release profile in the gastrointestinal environment, in particular a multicomponent dosage form comprising components in the form of either a body and a film means, or a capsule shell, linker and a film means, both forms being suitable for oral dosing.

## Description

The present invention relates to a pharmaceutical dosage form, being a multicomponent dosage form comprising components in the form of either a body and a film means, or a capsule shell, linker and a film means, both forms being suitable for oral dosing.

Various types of pharmaceutical dosage form are known for oral dosing. Pharmaceutical capsules are well known, generally being intended for oral dosing. Such capsules generally comprise an envelope wall of a pharmaceutically acceptable, e.g. orally ingestible, polymer material such as gelatin, although other materials for capsule walls, e.g. starch and cellulose based polymers are also known. Such capsules generally have soft walls made by making a film on a capsule former, which is then allowed to dry. Rigid walled capsules made by injection moulding are also known, see for example US 4576284, US 4591475, US 4655840, US 4738724, US 4738817 and US 4790881 (all to Warner Lambert). These disclose specific constructions of capsules made of gelatin, starch and other polymers, and methods of making them by injection moulding of hydrophilic polymer - water mixtures. US 4576284 specifically discloses such capsules provided with a cap which closes the capsule, and which is formed in situ on the filled capsule by moulding. US 4738724 discloses a wide range of rigid capsule shapes and parts.

Multi-compartment capsules, including those of the type where each compartment has different drug release characteristics or for example contains a different drug substance or formulation are also known, for example in US 4738724 (Warner-Lambert), US 5672359 (University of Kentucky), US 5443461 (AlzaCorp.), WO 9516438 (Cortecs Ltd.), WO 90/12567 (Helminthology Inst.), DE-A-3727894, BE 900950 (Warner Lambert), FR 2524311, NL 7610038 (Tapanhony NV), FR 28646 (Pluripharm), US 3228789 (Glassman), US 3186910 (Glassman) among others. US 4738817 discloses a multicompartment capsule with a similar construction to those of US 3228789 and US 3186910, made of a water-plasticised gelatin.

Another type of dosage form is disclosed in WO 01/08666 (SmithKline Beecham) wherein the dosage form comprises two or more capsule shells linked together via a linker unit having a plug part which extends into an open end of a shell.

Whilst the dosage form of WO 01/08666 provides advantages over other prior art dosage forms e.g. greater flexibility in producing a dosage form adapted to a patient's specific requirements, there nevertheless remains a need for alternatives. In particular there remains a need for a dosage form that is of a simple construction is easy to manufacture and that can provide a desired drug-release profile e.g. constituting both immediate and delayed release of a drug substance in a single dosage form. In particular it is an object of this invention to provide a dosage form that is easier to fabricate using ultrasonic welding to connect the components together. In the dosage form of WO 01/08666, to form the ultrasonic weld it is normally necessary to transmit ultrasonic energy along the whole length of the capsule shell to weld it to the linker. This can result in problems of shattering of the capsule shell, the need for a high power ultrasonic horn and handling capacity. It is an object of the invention to provide a dosage form that meets these requirements.

According to the invention there is provided a multicomponent pharmaceutical dosage form suitable for retaining drug substance which drug substance can be released to provide a controlled drug release profile in the gastrointestinal environment, characterised by a body having at least one cavity, said cavity having a mouth opening, and a film means connected to the body and closing the mouth opening.

By "controlled drug release" is meant that drug may be released from the dosage form to provide a defined, pre-determined drug release profile. Drug may be released from the dosage form at different rates, at different times following administration to the patient, or in different sites within the patient's gastrointestinal system. For example the dosage form may be designed to provide rapid release, immediate, modified release such as sustained or pulsatile release characteristics e.g. an immediate first pulse of drug followed by a delayed second pulse of drug at some later time point. Preferably the dosage form provides a pulsed release profile resulting in immediate release of a portion of the drug substance contained therein, typically within up to 15 minutes following ingestion of the dosage form, followed by a subsequent or delayed release of another portion of drug contained in the same dosage form, effected at some later point, typically up to 4 hours later. Usually immediate release takes place in the region of the stomach whilst delayed release occurs in another part of the gastro-intestinal compartment such as the small intestine or the gut.

Suitably the dosage form opens to release drug substance in the gastrointestinal environment. The process by which the dosage form opens may comprise one or more physical and/or chemical processes. For example the film means, the body, or a connection between the two such as a weld, may on exposure to a particular gastrointestinal environment and/or after a suitable time delay, shear or burst to release the content of a part or all of the dosage form. For example welded parts may separate as a consequence of differential swelling on hydration of the parts. Alternatively a component of the dosage form e.g. the film means, the body, a connection between the two, may dissolve partly or wholly or be otherwise breached to release the content of part or all of the dosage form.

Suitably the invention may comprise a plurality of cavities and/or a plurality of film means.

The present invention relates to a multi-component pharmaceutical dosage form for retaining a drug substance to provide a controlled drug release profile in the gastrointestinal environment, characterized by a first capsule shell comprising a first body (101) defining a first cavity (102) having a first mouth opening (103) and an opposite closed end (104); a second body (200) comprising a closure for the first mouth opening (103) and defining a second cavity (204) having a second mouth opening facing towards the closed end (104) of the first body (100), the second mouth opening being closed by a film means (206) between the first and second cavities connected to the second body (200)by a weld; and a second capsule shell (302) defining a further cavity (303) having a further mouth opening (304) and an opposite closed end (306), the second body (200) also comprising a closure for the further mouth opening ( 304) of the second capsule shell (302); wherein the first body (101), the second body (200) and/or the film means (206) are made of a polymer which dissolves or disintegrates in the environment of the upper or lower digestive tract, or which hydrates in the digestive tract, such that the dosage form opens to release the drug substance in the gastro intestinal environment.

In a embodiment the body may comprise a base wall having an upper surface, the first and second cavities being adjacent and defined by skirt walls extending upward from the upper surface of the base wall, the skirt walls terminating in a common rim defining the respective first and second mouth openings. Suitably the adjacent first and second cavities and their mouth openings are divided from each other by a dividing wall across the base wall and connected to the common skirt wall. Preferably the base wall is generally flat and the skirt wall and the dividing wall extend generally perpendicular to the upper surface of the base wall. A first and second film means may connect to the body to close both the first and second mouth openings of the first and second cavities respectively. Suitably the first and second film means are connected to the rim of the mouth openings.

In an embodiment comprising a first body and optionally second body, a first film means may close the mouth opening of the first cavity and the second cavity may be closed by a second film means or a second body connected- A -to the first body. Suitably the first film means may be connected to the rim of the mouth opening of the first cavity and second film means or the second body (when present) may connect to an exterior skirt wall extending upwards from the upper surface of the base wall of the first body.

In an embodiment the base wall may comprise a weakened portion such that release of drug substance contained in a first cavity in the vicinity of the weakened portion may be released in advance of drug being released from a second cavity which does not contain a base comprising a weakened portion. Advantageously the second embodiment allows drug to be filled in the dosage form in a single step operation.

In a embodiment the body defines a cavity having a longitudinal depth direction and the film means comprises a first and second film means, longitudinally spaced apart so as to define a cavity between the first and second film means and/or between the first or second film means and the base wall. Suitably the dosage form of this embodiment is substantially tub-shaped. For example the dosage form may comprise a body having a base wall having an upper surface and a skirt wall extending longitudinally upwardly therefrom to terminate in a rim defining the mouth opening. In this embodiment one cavity is generally located above the other. For example the mouth opening may be closed by a first film means and a second film means may be located under the said first film means to define an upper compartment between the first film means and the second film means. For example both the first and second film means may be connected to the rim of the skirt wall in the same vicinity. For example the first film means may be on top of the second film means, in contact with the second film means and connected to the second film means. Alternatively for example the first film means may be connected to the rim of the skirt wall whilst the second film means is longitudinally downwardly remote from the first film means e.g. the second film means may be connected to the skirt wall intermediate between the rim and the base wall so that the second film means is intermediate between the first film means and the base wall e.g. less than 50% of the depth or more than 50 % of the depth down. In the third embodiment the first film means may be substantially planar or blister convex relative to the second film means. The skirt wall may further comprise a ledge to accommodate the first and/or second film means. For example if the second film means is connected to the skirt wall intermediate between the first film means and the base wall a ledge may be provided intermediate between the first film means and the rim and the base wall.

In a embodiment there is provided a first body comprising a first cavity having a mouth opening and a second body comprising a second cavity having a mouth opening which bodies are connected to each other around respective rims of each mouth opening and a film means separates the first cavity from the second cavity. Suitably the first and second body may be arranged mouth-to-mouth i.e. one mouth facing upwardly and the other facing downwardly. A single film means may close both mouth openings or each mouth opening may be closed by a respective film means. Suitably each cavity contains drug substance as hereinbefore described. In one embodiment the film means and one body may comprise material to effect delayed release and the other body may comprise material to effect immediate release. An embodiment such as this provides a dosage form having modified release characteristics.

A dosage form of this embodiment may comprise a first body which comprises a first capsule shell defining a first cavity, e.g. a substantially cylindrical capsule shell, having a first mouth opening and an opposite closed end, a second body may comprise a closure for the first mouth opening, and the second body may define a second cavity having a second mouth opening facing toward the closed end of the first body when the second body is in place as a closure, and the second mouth opening is closed by a film means, such that the film means is between the first and second cavities. The second body may fit in a plug and socket relationship into the first mouth opening.

Such a second body may include one or more, preferably one, second cavity.

Such a dosage form may further include a second capsule shell defining a further cavity, e.g. a substantially cylindrical capsule shell, having a further mouth opening and an opposite closed end, and the second body may also comprise a closure for this further mouth opening of this second capsule shell. The second body may fit in a plug and socket relationship into the further mouth opening. For example the second body may have a first surface which when the second body is in place as a closure for the first capsule shell faces the closed end of the first capsule shell, and the second cavity is formed in the first surface, and an opposite surface which when the second body is in place as a closure for the first capsule shell faces the closed end of the second capsule shell. Therefore such a dosage form of this fourth embodiment may comprise a linear arrangement of:
a first capsule shell defining a first cavity and having a first mouth opening,
a second body fitting in a plug and socket relationship into the first mouth opening, the second body having a second cavity therein having a second mouth opening which is closed by a film means, such that when the second body is so fitted into the first mouth opening the film means is between the first and second cavities,
and a further capsule shell defining a further cavity and having a further mouth opening into which the second body fits in plug and socket relationship.

Typically the walls of such first and further capsule shells may have a wall thickness of 0.2-1.0 mm, preferably 0.3-0.5 mm.

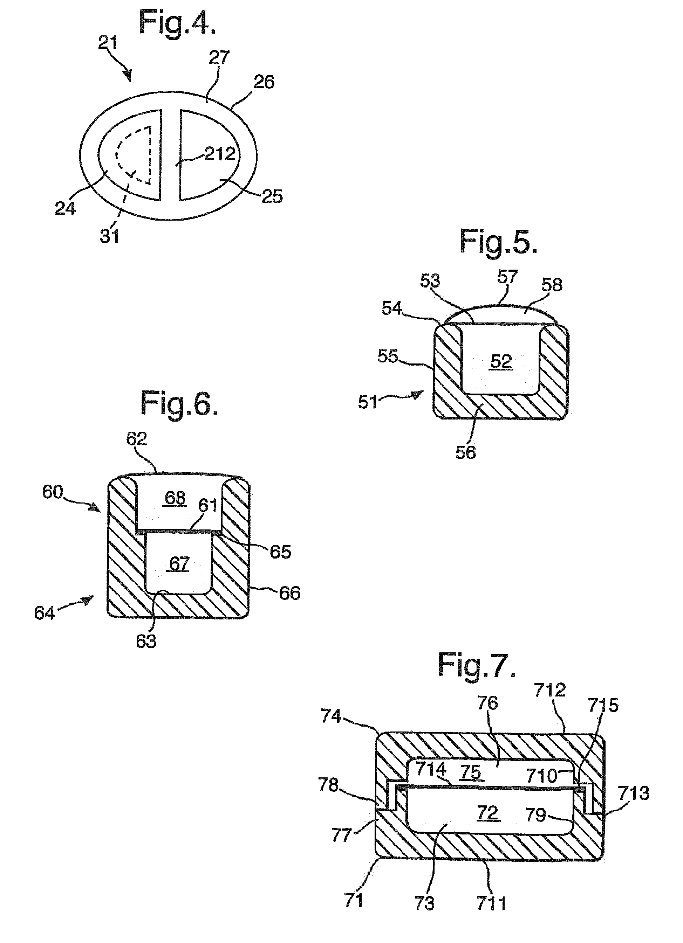

Suitably such first and second capsule shells may be slightly tapering, being narrower at their closed ends than at their mouth opening, to assist removal from a mould during manufacture. Suitably the mouth openings of the first and second capsule shells may be bounded by a rim, and the second body may engage with one or both of these first and/or second mouth openings in a plug-and-socket relationship. Suitably the second body may be attached, e.g. welded in place as a closure in the mouth opening(s) of the capsule shell(s), for example using an ultrasonic weld. For example the dosage form may have a construction generally similar to that disclosed in Figs. 6A-6C of WO 01/08666 and the related description, with the second body comprising a "linker" between the two capsule shells. Suitably the second mouth opening may be bounded by a rim, and the film means may be attached, e.g. welded in place around the second mouth opening, for example using an ultrasonic weld.

In these last-described dosage forms of the fourth embodiment a capsule shell may be made of an immediate or delayed/pulsed release polymer. If there are two capsule shells both may be immediate release, both may be delayed/pulsed release, or one may be immediate release and one delayed release. The film means closing the second mouth opening may be immediate or delayed/pulsed release polymer. For example the first and further capsule shells and the film means may comprise an immediate release polymer, and the first and second cavities may enclose respective drug substances intended for immediate release but which are best kept separate until release.

Advantageously the invention may provide a dosage form in a desired shape. For example the dosage form may be substantially cylindrical, which includes shapes which have a circular, oval or oblate circular cross section across the longitudinal axis, and shapes which have parallel or tapering e.g. with side walls which taper conically, which includes both true cones i.e. with straight side walls, and cones with curved side walls, over at least part of their extent. Dosage forms that are substantially elongated or circular when viewed in cross section across the longitudinal axis but being short in their longitudinal direction relative to their dimension across the cross section may resemble respectively capsules or tablets. Dosage forms that resemble tablets are preferred, e.g. longitudinally flattened cylinders, flattened spheroids or flattened ellipsoids.

Film means of the invention may be produced in a number of ways e.g. by hot-melt extrusion, solvent casting, or by an injection moulding process to produce, respectively, a continuous sheet / layer, or a series of discrete film parts. Preferably the film means is in the form of a sheet / layer that may be cut to size to fit the corresponding body. Film means may also be combined to form multi-laminated constructions of film means in which different polymer films are joined to create a sandwich-type layer with increased functionality compared to single layer films. Suitably the thickness of the film means is in the range 20-300 microns (µm) preferably 25-100 microns (µm). A body according to the invention may be produced by injection moulding processes which can enable the body to be made with precision. Suitable techniques of injection moulding are known for example from the art of manufacture of small plastic components. Processes such as those described in Cuff. G and Raouf. F, Pharmaceutical Technology, June 1998, p96-106, may be used to manufacture body parts. Consequently the invention also provides a moulding process for example an injection moulding or powder compression process wherein the body part of the dosage form is made in a mould cavity. Preferably the moulding process is a hot runner process. The invention also provides a mould or die, suitable for use in the moulding process. Such a mould or die may have a mould cavity corresponding to the shape of the body. Moulds may be made by known metal engraving processes such as spark erosion and it is generally preferred to use moulds made from pharmaceutically acceptable metals e.g. steels of the type known for use in tablet compression dies.

In an assembled dosage form of the invention, the component parts of the dosage form namely the film means and the body may be connected together e.g. by a weld such as a thermal weld, an ultrasonic weld, inductive weld or an adhesive weld (e.g. curable adhesives such as UV curable adhesive). A thermal weld may for example be achieved by bringing the film means and the body into adjacent contact and applying localised heating for example produced by directing a laser beam or a fine jet of hot gas e.g. nitrogen at the area where the film means and the body are in contact. In thermal, inductive and ultrasonic welding normally localised fusion together of the materials of adjacent parts of the dosage form which are in contact occurs, and on subsequent solidification of the materials a bond is formed between the adjacent parts. An adhesive weld may be achieved by applying an adhesive to at least one part of the dosage form which when the dosage form is assembled is in contact with another part, and then causing or allowing the adhesive to set.

The multicomponent dosage form of the present invention is particularly suited to fabrication using ultrasonic welding. Ultrasonic welding is a known technique involving the use of high frequency sound energy to soften or melt a thermoplastic material at the site where a joint with the material is required. A general description of ultrasonic welding is for example to be found in the publication "Ultrasonic Welding of Thermoplastics" (TWI Ltd., Abington, Cambridgeshire GB, (1997)). Parts to be joined are held together under pressure and then subjected to ultrasonic vibrations usually at a frequency of 20 - 40 kHz. The actual mechanism responsible for the generation of heat at the joint site is not well understood. An ultrasonic welding machine comprises five main components, being a power supply, a control system, a welding head, fixturing to hold the parts to be welded, and a system to apply the required pressure. The power supply converts electricity into high frequency electric power which drives a transducer, e.g. a piezoelectric transducer, which converts electrical energy, e.g. from the mains supply, into mechanical, i.e. ultrasonic, energy. Between the transducer and the parts to be welded is located a booster and horn system, being a usually metallic component which serves to amplify the ultrasonic waves (the booster horn), transmit the clamping pressure, and deliver the sound energy to the part to be welded (the sonotrode or welding horn). For successful ultrasonic welding careful design of the parts to be welded and set up of the welding equipment is important. The rim of the mouth opening may be profiled to facilitate formation of an ultrasonic weld.

It will be apparent to those skilled in the art how a mouth opening of a cavity may be made of a suitable size and profile to facilitate the welding of a film means to the rim to close the mouth opening. One suitable profile of the rim of the mouth opening to facilitate ultrasonic welding of the film means thereto may comprise a rim in the form of an upwardly facing generally flat surface generally perpendicular to the depth direction of the cavity. Optionally such a surface may be bounded by a kerb edge e.g. a small upwardly extending wall around the outer periphery of the rim. The film means may be placed on top of such a surface with one side of the film in contact with the surface, and by applying the ultrasonic horn to the film means on the opposite side of the film, the weld may be formed. There may be one or more small upwardly extending ridges on the surface which may collapse on application of the horn to the weld. Additionally or alternatively the rim may have a concavity such as a groove therein into which the film means e.g. an edge thereof, may fit.

Alternatively the rim may be flat and the ultrasonic horn may have one or more small upwardly extending ridges on the surface acting on the film in order to focus sound energy on a specific point or points to achieve welding of the two components. It is preferred to use an ultrasonic hom which can both form the weld and cut the film means to a suitable size and shape corresponding to the mouth opening and its rim.

The film means and body of a dosage form of the invention are made of materials having particular physico-chemical characteristics to achieve a desired release profile. The film means and the body may be comprised of material designed to provide rapid dissolution, immediate dissolution and/or modified dissolution e.g. delayed dissolution to confer sustained or pulsatile release characteristics, and combinations thereof. For example in an embodiment according to the invention e.g. in the first embodiment herein described, the first film means may be designed to provide rapid or immediate dissolution and the second film means may provide modified dissolution such as sustained or pulsatile release characteristics. In an alternative arrangement of the first embodiment the first film means may be designed to provide rapid or immediate dissolution and the body may be designed to provide modified dissolution. In an alternative embodiment, such as a variant of the second embodiment, the film means may be designed to provide modified dissolution and the body, e.g. in the vicinity of the weakened portion may provide rapid or immediate dissolution.

Suitable materials for the provision of a desired release profile include pharmaceutically acceptable polymers and blends thereof which may be injection moulded to form a body such as a capsule shell or linker, and may also be made into thin films to provide the film means of the dosage form. If the component parts of the dosage form, e.g. body and film means are to be welded together e.g. by an ultrasonic weld then preferably polymers or blends thereof are selected for the respective parts to be welded which are compatible to encourage weld formation. For example the polymer of each of such components may be blended with a common polymer to facilitate this.

For purposes herein representative examples of polymers suitable for injection molding or film-formation into multicomponent dosage forms and for use in pharmaceutical applications, include, but are not limited to, poly(ethylene) oxides (PEO), polyethylene glycol's (PEG), mixtures of PEG's and PEO's, polyvinyl alcohol (PNA), polyvinyl acetate, povidone (polyvinyl pyrrolidone), cellulose derivatives such as carboxymethyl cellulose, methyl cellulose, ethylcellulose, hydroxyethyl cellulose, hydroxypropylcellulose, hydroxyethyl mefhylcellulose, hydroxy propylmethyl cellulose (HPMC) e.g. those sold under the tradename Klucel, , hydroxypropylmefhyl cellulose phthalate, cellulose acetate phthalate, noncrystalline cellulose, starch and its derivatives such as hydroxyethyl starch, sodium starch glycolate, natural polymers (such as polysaccharides like pullulan, carrageenan, xanthan, chitosan or agar gums), polyacrylates and poly (meth)acrylates, and its derivatives such as the Eudragit family of polymers available from Roehm Pharma, poly(alpha-hydroxy acids) and its copolymers such poly(caprolactone), poly(lactide-co-glycolide), poly(alpha-aminoacids) and its copolymers, polyglycolysed glycerides (such as Gelucire® 44/14, Gelucire® 50/02, Gelucire® 50/13 and Gelucire® 53/10), carboxyvinyl polymers (such as Carbopols), and polyoxyethylene-polyoxypropylene copolymers (such as Poloxamer 188™); and combinations or mixtures thereof.

Also potentially suitable for use herein are the polymers poly(orthoesters), polyphosphazenes, poly(phosphoesters), and polyanhydrides, and combinations or mixtures thereof.

Additionally, hyaluronic acid, alginates, carragenen, collagen, gelatin, and albumen may also be suitable for injection moulding herein, either alone or in combination with another polymeric blend. It is recognised that the ultimate choice of polymers if not previously approved by the regulatory agencies of the world, are in the category of generally recognised as safe (GRAS) approved.

Especially suitable polymers for use in the invention are those that preferentially dissolve or disintegrate at a defined point or region in the digestive tract.

For example a polymer may dissolve or disintegrate in the environment of the upper digestive tract or the stomach, and so be termed an "immediate" release polymer, for example dissolving or disintegrating within ca. 1 hour, e.g. within ca. 30 minutes of oral administration. For example a polymer may dissolve or disintegrate in the environment of the lower digestive tract e.g. the intestine, and so be termed a "delayed" or "pulsed" release polymer, for example substantially not releasing any medicament contained therein until the polymer has reached the intestine. Such polymers include the known acrylic and/or methacrylic acid-based polymers which are soluble in intestinal fluids e.g. the Eudragit series of polymers, produced by Roehm GmbH in Germany. Examples of Eudragit polymers include Eudragit E e.g. Eudragit E100, which preferentially dissolves in an acidic, e.g. up to pH 5, environment e.g. in the acidic environment of the stomach, or enteric polymers such as Eudragit L, e.g. Eudragit L100, and/or Eudragit S, which preferentially dissolve in a more alkaline pH environment e.g. in the environment of the intestine. Suitably the polymer Eudragit 4135F dissolves only above pH7 e.g. in the colon and so is suitable for formulation as a delayed release or pulsatile release component. However this polymer may be formulated so as to be pH independent, as discussed herein below. Other suitable polymers also include polymers which are insoluble but hydrate at a controlled rate e.g. at a predetermined rate in the digestive tract such as Eudragit RL e.g. Eudragit RL 100 and/or Eudragit RS e.g. Eudragit R100 and/or blends of Eudragit polymers.

The Eudragit polymers i.e. poly(meth) acrylate copolymers, such as Eudragit 4135F, Eudragit E100 and Eudragit L100 are included as preferred polymers for use in forming the component parts of a dosage form according to the invention namely one or more of a body, a film means, a capsule shell and/or a linker. A particular polymer disclosed in US 5,705,189, Emulsion E2 (column 6 line 10), being a copolymer of methacrylic acid, methyl methacrylate and methyl acrylate, suitably in a ratio of 10:25:65, is a preferred polymer for use in the present invention (as are the polymers disclosed in WO 01/43935 and WO 01/39751, both in the name of Roehm). This ratio of components is also known as Eudragit 4135F and is a solid product obtained from Eudragit FS30D, and as noted above is available from Roehm Pharma, Germany.

Eudragit E100 comprises butylmethacylat-(2-dimethylaminoethyl) methacrylat-methylmethacylat-copolymer (1 :2:1) and is a copolymer based on (2-dimethylaminoethyl)methacrylalate, butyl methacrylate and methylmethacrylate, typically in a 2:1 :1 ratio, having a mean molecular weight of about 150,000. It contains not less than 20.8 and not more than 25.5% dimethylaminoethyl groups in the dry substance. Eudragit L100 comprises polymethacrylic acid, methyl methacrylate) in a ratio of 1 : 1.

Suitably a delayed release or pulsed release body, film means, capsule shell or linker comprises a blend based on Eudragit 4135F, or a blend based on Eudragit L100. Suitably an immediate release body, film means, capsule shell or linker comprises Eudragit E100, RL100, RS100, hydroxypropylcellulose, or a blend based thereon. A preferred combination of polymers for use in any film means/body combination comprises Eudragit E100 or hydroxypropylcellulose for immediate release, and Eudragit E4135F and/or Eudragit L100 for delayed or pulsed release.

When the delayed release or pulsed release polymer comprises Eudragit 4135F, this polymer is suitably blended with at least one lubricant and at least one dissolution modifying agent to achieve quality, non-distortion moulded components which readily release from injection moulds. Examples of suitable dissolution modifying agents, lubricants and other optional additional additives or excipients are provided below. Suitably Eudragit 4135F is used in an amount of about 20 to about 90% w/w , preferably from about 50 to 90%w/w, the dissolution modifying excipient is used in an amount of about 2.5 to about 30% w/w, and the lubricant in an amount of about 5 to about 30%w/w. A suitable polymer blend comprises a polymer e.g. Eudragit 4135F ca. 78%w/w, a lubricant e.g. stearyl alcohol ca. 12%w/w, and a dissolution modifying agent e.g. hydroxypropylmethylcellulose ca.10%w/w. Suitable blends based on 4135F are for example disclosed in WO 02/060384. When the delayed release or pulsed release polymer comprises Eudragit L100, the polymer is preferably blended with at least one lubricant and at least one plasticizer. Suitably the polymer is used in an amount of about 40 to about 70%w/w, the lubricant is used in an amount of about 2 to about 10%, and the plasticizer is used in an amount of about 20 to about 50% w/w. A suitable polymer blend comprises a lubricant e.g. glyceryl monostearate ca. 5%, a plasticizer e.g. triefhyl citrate ca.40% and Eudragit LI 00 up to 100%w/w.

Alternatively the delayed release polymer may comprise a graft copolymer comprising PVA and PEG. One commercially available form of such a polymer comprises 75% PNA and 25% PEG and is available under the trade name Kollicoat IR.

When the immediate release polymer comprises Eudragit E100, the polymer may be used either by itself or as a blend. Suitably a blend may comprise one or more dissolution modifying agent(s) and one or more lubricant(s). Suitably the polymer is used in an amount of about 30 to about 90% w/w, the dissolution modifying excipient is used in an amount of about 0 to about 70%w/w e.g. in the range 5 to 70%w/w, and the lubricant is used in an amount up to about 30%w/w. Preferably the polymeric blend further comprises a plasticiser in the range from 0% to about 5% and a processing agent in the range 0 to 30%w/w. A suitable polymer blend comprises a polymer e.g. Eudragit E100 ca 53%, a lubricant e.g. stearyl alcohol ca. 12%, a processing aid e.g. polyethylene oxide ca. 20%, a strengthening aid e.g. talc ca. 10% and co-povidone ca. 5%. Suitable blends are for example disclosed in WO 02/060385.

An immediate release polymer may comprise a hydroxyethyl cellulose, low molecular weight hydroxypropylcellulose and/or a low-substituted hydroxypropyl cellulose. Further, two or more polymers may be used in combination to form blends having the desired characteristics. For example it is known from WO02/060384 that a novel blending of components has the ability to render the poly(methacrylates), such as Eudragit 4135F, which are pH dependent independent of this characteristic. A suitable immediate release or pulsed release blend may comprise one or more cellulose derivatives in an amount from about 20-80%w/w e.g. hydroxypropylcellulose derivatives of varying molecular weights available commercially as Klucel e.g. Klucel EF and Klucel JF, having a molecular weight respectively of ca. 80000 and ca. 140000, each being present in an amount ca. 32%, 5-30% of a lubricant e.g. stearyl alcohol ca. 12%w/w, and 20-90%w/w of a polymer e.g. ca. 23‰ Eudragit 4135F. Preferably such a blend is used in a capsule shell according to the invention. When welded to a delayed release component based on Eudragit 4135F as described above, such a weld may separate as a result of differential swelling on hydration.

It is found that a 20-300 micron (µm) thick film of the above-mentioned immediate release polymers can dissolve in less than 5 minutes in the environment of the digestive tract. However if a film based on one or more such immediate release polymers is to be welded e.g. ultrasonically to a body made of a different polymer such as a polymer or polymer blend containing a Eudragit polymer, it is preferable to blend the polymer of the film with a polymer included in the body it is to be welded to.

The aforementioned polymer-based body/ film means combinations, and capsule shell/linker combinations may be used in any one of the dosage forms according to the embodiments of the invention. For example a dosage form according to the first embodiment may comprise a body comprising Eudragit 4135F and a film means comprising Eudragit E100 and/or Eudragit L100. For example the body may comprise Eudragit 4135F and a first and second film means may comprise Eudragit E100 or Eudragit L100 respectively, or alternatively a first film means may comprise Eudragit E100 and a second film means may comprise Eudragit L100.

In general, for use herein, suitable dissolution modifying excipients include a disintegrant such as sodium starch glycollate e.g. Explotab, cross-linked PNP e.g. Kollidon-CL, copovidone e.g. KoUidon VA 64 or starch e.g. starch 1500; a swelling agent such as polyvinylpyrrolidone (PVP, also known as povidone) e.g. ISP-plasdone or BASF-Kollidon and primarily grades with lower K values e.g. K-15, K25 and K-30; a cellulosic derivative such as hydroxypropylme hylcellulose; a wicking agent such as a low molecular weight solute e.g. mannitol, lactose and starch; inorganic salts such as sodium chloride (typically at 5-10%). Suitable lubricants or glidants include stearyl alcohol, stearic acid, glycerol monostearate (GMS), talc, magnesium stearate, silicon dioxide, amorphous silicic acid, fumed silica and combinations thereof. Suitable plasticisers include triethyl citrate (TEC), triacetin, tributyl citrate, acetyl triethyl citrate (ATEC), acetyl tributyl citrate (ATBC), dibutyl phthalate, dibutyl sebacate (DBS), diethyl phthalate, vinyl pyrrolidone glycol triacetate, polyethylene glycol, polyoxyethylene sorbitan monolaurate, propylene glycol, or castor oil; and combinations or mixtures thereof. The choice of polymer will determine which plasticiser is suitable for use and this will be apparent to the man skilled in the art. For instance, triacetin is not preferred for use with E100 or 4135F at levels of about 5% but may be suitable for use with Eudragit RS or RL, or PVA.

The dosage form of the invention may be wholly or partly coated with a polymeric coating, such as the Opadry film coating system, e.g. to influence its release characteristics and/or to protect an otherwise fragile film means and/or to modify the appearance of the dosage form. A coating may comprise a delayed or pulsed release polymer which dissolves or is otherwise be breached in an environment of defined pH, such as that of the stomach or intestine, such as the Eudragit polymers discussed above. A typical such polymer is the enteric polymer Eudragit L30D 5S which dissolves in the intestine. For example a capsule shell made of an immediate-release polymer as described above may be coated with such a coating. Processes are well known in the art for applying such a coating to a substrate, and which are applicable to a component of a dosage form. Generally it may be preferable to apply such a coating to the components of the dosage form, e.g. the first or second body, capsule shells etc. before filling with a drug substance, because typical processes for applying such coatings involve heating the substrate to an elevated temperature, which may be detrimental to a drug substance contained therein.

The dosage form is particularly suitable for presentation as an oral dosage form containing one or more drug substances e.g. containing a combination of drug substances. When there is more than one cavity present each cavity may contain the same or different drug substance which may be released at the same time or a different rate or time after administration or place in the patient's gastro-intestinal system. Additionally or alternatively each of two or more cavities may contain respective substances which are incompatible in contact with each other prior to administration to a patient. Advantageously the invention provides a dosage form having variable drug content and/or drug release characteristics to provide a dosage form tailored to specific administration requirements. The drug substance(s) contained in the cavity(ies) may be present in any suitable form e.g. in the form of powder, pellets, granules, semisolids or liquids.

Dosage forms of the invention may be prepared by various processes. A typical process for making a dosage form comprising two cavities comprises the following steps:
1. Forming a body e.g. by injection moulding of a suitable polymer and forming a film means e.g. by hot-melt extrusion.
2. Optionally applying a polymer coat to the body.
3. Filling a first or second cavity with drug substance.
4. Closing the so-filled cavity with a film means.
5. Filling a second or first cavity with the same or different drug substance.
6. Closing the so-filled cavity.

When the dosage form comprises a body having two oppositely facing i.e. upwardly and downwardly facing mouth openings it is preferred to perform steps 3 and 4 on the cavity which is facing upwardly then to invert the body so the other cavity is facing upwards, then perform steps 5 and 6.

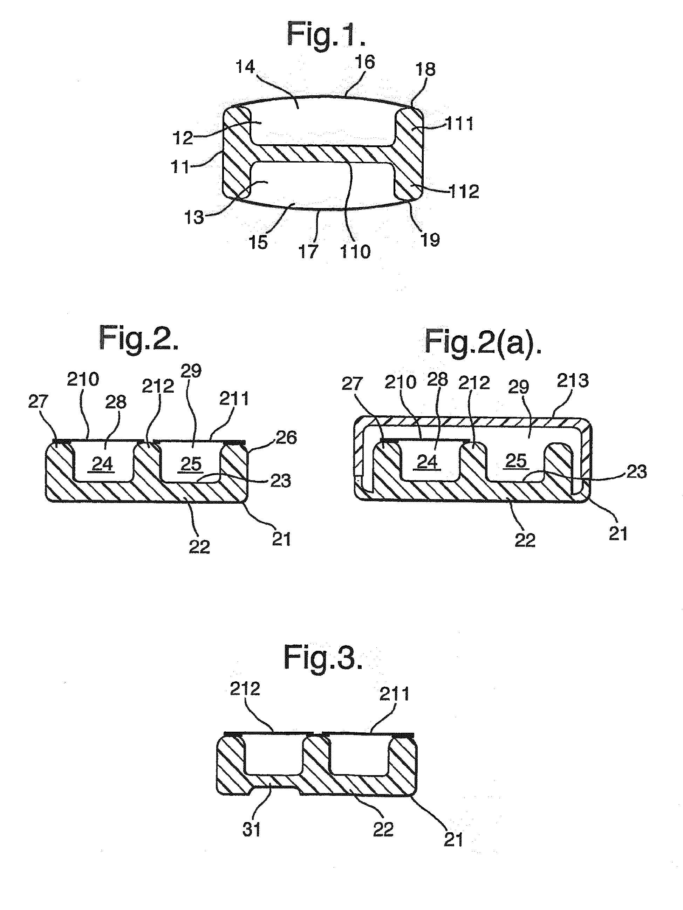

In an alternative aspect of the invention there is also provided the component parts of the invention e.g. a body for use in a dosage form according to the invention and comprising at least one cavity having a mouth opening and adapted for connecting a polymer film thereto. Details of the dosage forms referred to above will now be described with reference to Figs. 1-12 which show longitudinal plans and perpendicular views of a dosage form of the invention and/or components thereof.

Referring to Fig. 1 a dosage form is shown having an body 11, H' -shaped in longitudinal section comprising a first cavity 12 and a second cavity 13, the first and second cavities having a first and second mouth opening, 14 and 15, respectively. The body 11 is made of a delayed dissolution polymer, by injection moulding. The first and second mouth openings 14 and 15 are closed by first and second film means 16 and 17 respectively connected by an ultrasonic weld to the respective rim 18, 19 of the mouth opening 14, 15. The first and second film means 16 and 17 form a blister convex relative to the respective first and second cavity. The first film means 16 may comprise material that confers delayed release characteristics upon the dosage form e.g. Eudragit 4135F, whilst the second film means 17 may comprise material that confers immediate release e.g. Eudragit E100. The body comprises a base wall 110 and skirt walls 111,112 extending respectively upwardly and downwardly from the base wall 110 to define the cavities 12,13. The cavities 12 and 13 contain drug substance (not shown) and, if desired, any pharmaceutically acceptable carrier.

Referring to Fig. 2 a dosage form is shown comprising a body 21, generally 'w' shaped in cross section having a base wall 22 having an upper surface 23 with a first and second cavity, 24 and 25, being defined by a skirt wall 26 extending from the upper surface 23 and terminating in a common rim defining a first and second mouth opening 28 and 29. A first and second film means, 210 and 211, which are generally planar seal the first and second mouth openings 28 and 29. The first and second cavities 24 and 25 are divided from each other by a dividing wall 212 across the base wall 22. The first and second film means 210 and 211 may comprise material that confers immediate release and the body may comprise material that confers delayed release. A single common film (210,211) may close both mouth openings 28, 29.

Referring to Fig 2A a variation of the dosage form of Fig. 2 is shown, and corresponding parts are numbered correspondingly. A second body 213 is connected to a first body 21.

Referring to Fig. 3 a dosage form is shown disclosing a variant of the dosage of Fig. 2 in which the base wall 22 comprises a weakened portion 31 in the form of a thinned region of the base wall 22. The weakened portion 31 may comprise an immediate release material and the film means 210 and 211 may comprise a delayed release material.

Fig. 4 shows the dosage form of Figs. 2 and 3 in plan, without the film means.

The shape and position of thinned region 31 is also shown dotted.

Referring to Fig.5 there is disclosed a dosage form comprising a generally 'U'-sectioned shaped body 51 in cross section having a first cavity 52. A first film means 53 closes the first cavity 52 at the rim 54 of a skirt wall 55 extending upwardly from the base wall 56 of the body 51. A second film means 57, located above the first film means 53 defines a second cavity 58. Both cavities 52 and 58 contain drug substance (not shown). The first film means 53 may form a planar or concave blister relative to the substantially planar base wall 56. The second film means 57 may form a planar or convex blister (as shown) relative to the substantially planar base wall 56. Second film means 57 is situated above and in contact with first film means 53 and is connected thereto.

Referring to Fig. 6 a dosage form 60 is shown being a variant of the dosage form of Fig. 5. In this embodiment the first film means 61, is located between the second film means 62 and the base wall 63 of the body 64. The body 64 has a ledge 65 which extends inwardly from the skirt wall 66 and which serves as a means for supporting and providing a connecting point between the first film means 61 and the body 64. First and second cavities 67, 68 are thereby defined.

Referring to Fig. 7 a dosage form is shown comprising a first body 71 having a first cavity 72 having a mouth opening 73 and a second body 74 comprising a second cavity 75 having a mouth opening 76. The bodies 71, 74 are connected to each other around the respective rims 77 and 78 of skirt walls 79 and 710, extending respectively upwardly and downwardly from base walls 711, 712, of the first and second bodies 71 and 74 at weld 713. A film means 714 is welded at 715 to skirt wall 79 of body 71 to close mouth opening 73. Each cavity 72 and 75 contain drug substance (not shown). The first body 71 may comprise immediate release material and the second body 74 and the film means 714 may comprise delayed release material.

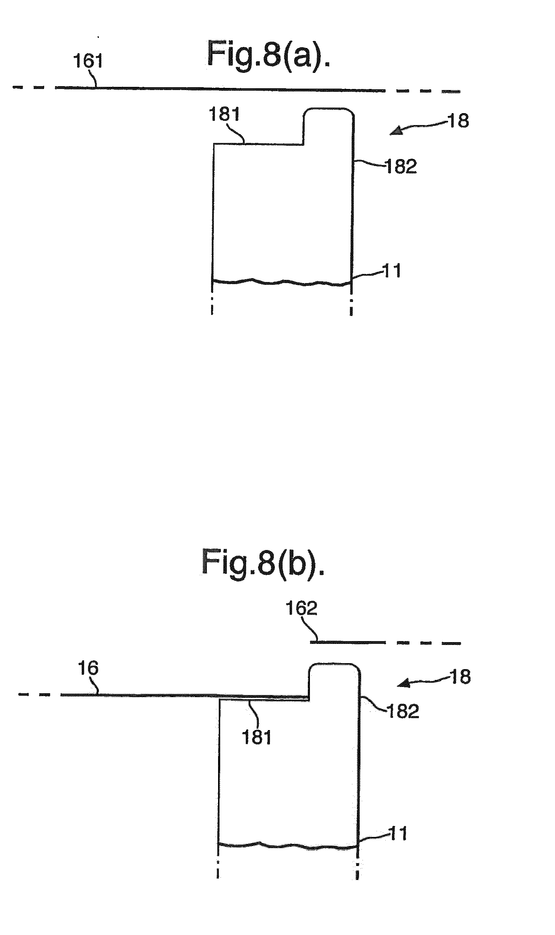

Fig. 8A and Fig 8B, show a construction of the rim 18 of a dosage form according to Fig.1, suitable for forming an ultrasonic weld. The rim 18 comprises a generally flat upwardly facing surface region 181, bounded at its outer periphery by a small upstanding kerb 182. A small upstanding ridge (not shown) may be provided on flat surface 181 to facilitate formation of the weld. A film means 161 is welded to the surface 181 by positioning a sheet 161 of the film material above the rim 18 as shown in Fig. 8A, then bringing down an ultrasonic horn (not shown) on to the sheet 161 to compress and weld the sheet 161 material against surface 181, and simultaneously to cut the sheet material 161 at edge 162. As shown in Fig 8B the film means 16 is thereby connected by a weld to the body 11 and by the kerb 182.

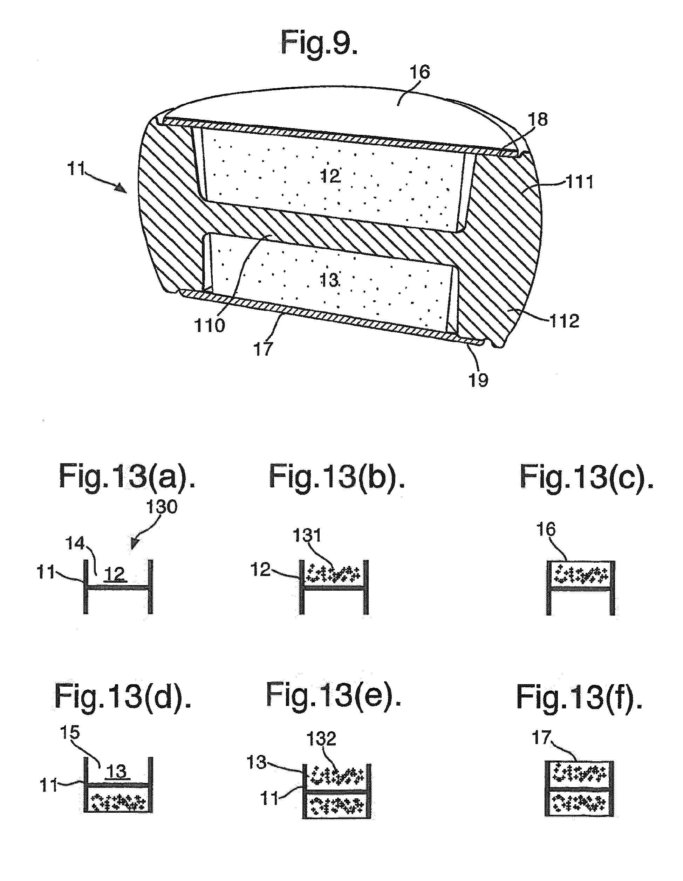

Fig. 9 shows a perspective view of a dosage form according to Fig.1.

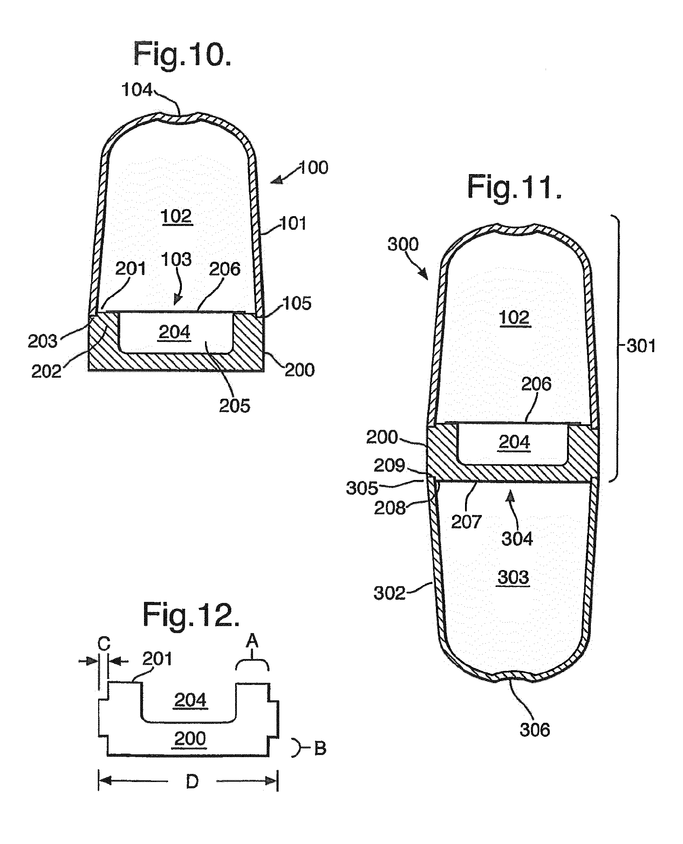

Referring to Fig. 10 a dosage form 100 is shown in longitudinal section. This dosage form comprises a first body 101 which comprises a capsule shell defining a first cavity 102. The capsule shell 101 is substantially cylindrical and has a mouth opening 103 and an opposite closed end 104. The capsule shell 101 is slightly tapering, being narrower at its closed end 104 than at its mouth opening 103. The mouth opening 103 is bounded by a circular rim 105.

A second body 200 is provided, comprising a closure for the first mouth opening 103. The second body 200 is of generally cylindrical shape and has a surface 201 which is formed into a plug part 202 generally conforming to the inner profile of the mouth opening 103 and which can fit snugly in a plug-socket relationship into the mouth opening 103 of the capsule shell 101, surrounded by a ledge 203. The rim 105 and the plug part 202 are both profiled in a manner similar to that disclosed in Figs. 6A, 6B and 6C of WO 01/08666 and the corresponding description, to facilitate the formation of an ultrasonic weld between the rim 105 and plug part 202 and/or ledge 203 using a process as disclosed in WO 01/08666.

A second cavity 204 is provided in the second body 200, in the form of a generally cylindrical concavity in the body 200, although cavity 204 may be of any other convenient shape. Cavity 204 has a second mouth opening 205 facing toward the closed end 104 of the first body 101 when the second body 200 is in place as a closure as shown.

The second mouth opening 205 is closed by a film 206, which in the construction shown is between the first and second cavities 102, 204. The mouth opening 205 is bounded by a rim, and the film 206 is attached around the rim of the mouth opening 205 by an ultrasonic weld between the surface 201 and the film 206. The film 206 may be welded in this way around the rim of the mouth opening 205 using known technology, e.g. using a sonotrode (term of the art) with a ring shaped tip with a diameter larger than the diameter of the opening 205.In use the capsule shell 101 and film 206 may be made of an immediate-release polymer, and the body 200 may also be made of an immediate-release polymer, or be made of an insoluble, slowly soluble or delayed/pulse release polymer. In such a construction the first and second cavities 102, 203 may respectively contain different substances which prior to administration should not be in contact, e.g. a respectively a drug substance and a pH adjuster such as a solid acid, so that mixing only occurs when the capsule shell 101 and film 206 are breached after oral administration.

Referring to Fig. 11 a further dosage form 300 is shown in longitudinal section. This dosage form comprises a first and second body, shown generally 301 which is identical in construction to that shown in Fig. 10, except that the surface 207 of the second body 200 opposite surface 201 is formed into a second plug part 208 and a second surrounding ledge 209.

The dosage form 300 includes a further capsule shell 302 of generally similar tapering cylindrical shape to the shell 101, having a mouth opening 304 bounded by rim 305, and an opposite closed end 306, and defining a further cavity 303. The second body 200 also acts as a closure for the mouth opening 304 of this further capsule shell 302, with plug part 208 fitting snugly into mouth opening 304. The plug parts 202, 208 have a suitable profile and dimensions, which can be determined experimentally, so that plug parts 202 and 208 fit snugly into mouth openings 103, 304

When in place as shown the rim 305 of the mouth opening 304 may be ultrasonically welded in place in contact with plug part 208 and ledge 209 in a manner similar to the dosage form of Fig. 10. In the construction shown in Fig. 11 the second body 200 comprises a "linker" between the two capsule shells 101, 302. Although in Fig. 11 the two capsule shells 101, 302 are shown as similar in size they may of course be different in size as a consequence of different quantities of substances contained in cavities 102, 303.

Referring to Fig. 12 some suitable dimensions for the second body 200 are found to be as follows. The overall cylindrical diameter D is ca 7.5mm. The width A of the flat ringshaped rim surface is 1.0 - 1.5 mm to achieve a suitable weld with the film 206. The height B of the plug part is 0.5 - 0.6 mm and the width C of the step around the base of the plug part is typically approximately the same as the wall thickness of the capsule shell 101 or 302, e.g. typically ca. 0.3 mm. The cavity 204 is typically ca. 4 - 4.5 mm diameter and 1-2 mm deep.

The dosage form 300 shown in Fig. 11 therefore comprises a linear arrangement of the first capsule shell 101, the second body 200 and the further capsule shell 302, with the second body acting as a linker between the first capsule shell 101 and the further capsule shell 302.

For example in use the capsule shells 101 and 302 may be made of an immediate-release polymer such as a Eudragit E1 00 or hydroxypropylcellulose blend. The body 200 may be made of a delayed/pulse release polymer such as a Eudragit 4135F blend, and the film may be an immediate release polymer comprising hydroxyethylcellulose typically 20-300 micron (µm) thick.

In such a construction the first and second cavities 102, 203 may respectively contain different substances which prior to administration should not be in contact, e.g. a respectively a drug substance and a pH adjuster such as a solid acid, so that mixing of these substances only occurs when the capsule shell 101 and film 206 are breached after oral administration. The cavity 303 may contain a further substance, eg. a drug substance, which is only released for example when the assembly of shell 302 and body 200 reaches the intestine after oral administration.

The cavities 102, 204, 303 may have a volume suitable to contained a desired quantity of drug substance contents. For example the cavities 102, 303 may each be suitable to contain ca. 250mg of a particulate substance, and the cavity 204 may be suitable to contain ca. 2 mg of a substance.

The capsule shells 101, 302 may be coated with a coating of an enteric polymer (not shown) such as Eudragit L30D 5S. The dosage forms 100, 300 may be filled and assembled as follows. Capsule shells 101, 302 are provided, and in an optional step one or both of capsule shells 101, 302 is/are coated with an enteric polymer such as Eudragit L30D 5S using a standard coating procedure. Second body 200 is provided, with its mouth opening 205 facing upward, and a substance is introduced into cavity 205. The film 206 is welded in place in surface 201. Capsule shell 101 is provided with its mouth opening 103 facing upward and a substance is introduced into cavity 102. Second body 200 is then provided with the surface 201 facing downward, and body 200 is fitted into place as a closure into mouth opening 103. A sonotrode may then be applied to either closed end 104 or surface 207 so that ultrasonic energy is communicated to the point of contact between rim 105 and ledge 203. Alternatively a sonotrode may be applied in the horizontal direction as shown to the outer surface of the rim 105 immediately adjacent to this point of contact. If required, the capsule shell 302 is provided with its mouth opening 304 facing upward, and a substance is introduced into cavity 303. The assembly of shell 101 and second body 200 is then provided with the second body 200 lowermost, and the body 200 is fitted into place as a closure into mouth opening 304. A sonotrode may then be applied to closed end 306 so that ultrasonic energy is communicated to the point of contact between rim 305 and ledge 209. Alternatively a sonotrode may be applied in the horizontal direction as shown to the outer surface of the rim 305 immediately adjacent to this point of contact.

Fig. 13 shows how a dosage form 130 according to the first embodiment, i.e. Figs. 1, 8 and 9 may be filled. As seen in Fig. 13A a body 11 is provided, oriented so that its first cavity 12 with its mouth opening 14 is facing upwardly. As seen in Fig. 13B drug substance 131 is introduced into cavity 12 by a suitable filling means (not shown). As seen in Fig 13C the mouth opening 14 is closed by a film means 16 ultrasonically welded thereto in a manner as described above. As seen in Fig. 13D the dosage form is inverted so that the body 11 is oriented so that its second cavity 13 with its mouth opening 15 is facing upwardly. As seen in Fig. 13E drug substance 132 is introduced into cavity 13 by a suitable filling means (not shown). As seen in Fig 13F the mouth opening 15 is closed by a film means 17 ultrasonically welded thereto in a manner as described above. The so-made dosage form 130 may now be further processed e.g. coated.

## Claims

1. A multi-component pharmaceutical dosage form for retaining a drug substance to provide a controlled drug release profile in the gastrointestinal environment, **characterized by** :
a first capsule shell comprising a first body (101) defining a first cavity (102) having a first mouth opening (103) and an opposite closed end (104);
a second body (200) comprising a closure for the first mouth opening (103) and defining a second cavity (204) having a second mouth opening facing towards the closed end (104) of the first body (100), the second mouth opening being closed by a film means (206) between the first and second cavities connected to the second body (200)by a weld; and
a second capsule shell (302) defining a further cavity (303) having a further mouth opening (304) and an opposite closed end (306), the second body (200) also comprising a closure for the further mouth opening ( 304) of the second capsule shell (302);
wherein the first body (101), the second body (200) and/or the film means (206) are made of a polymer which dissolves or disintegrates in the environment of the upper or lower digestive tract, or which hydrates in the digestive tract, such that the dosage form opens to release the drug substance in the gastro intestinal environment.

2. A multi-component pharmaceutical dosage form according to claim 1, **characterized in that** the weld is an ultrasonic weld.

3. A dosage form according to claim 1, **characterized by** a linear arrangement of:
the first capsule shell (101); the second body; and
the second capsule shell (302);
wherein the second body (200) fits in a plug and socket relationship into the first mouth opening (103) and; the further mouth opening (304).

4. A dosage form according to claim 3, **characterized in that** at least one capsule shell (101, 302) is made of a polymer which dissolves or disintegrates in the environment of the upper digestive tract.

5. A dosage form according to claim 4, **characterized in that** both capsule shells (101, 302) are made of said polymer.

6. A dosage form according to claim 1, **characterized in that** the film means (206) closing the second mouth opening is made of a polymer which dissolves or disintegrates in the environment of the upper digestive tract.

7. A dosage form according to claim 1, **characterized in that** both capsule shells (101, 302) and the film means (206) are made of a polymer which dissolves or disintegrates in the environment of the upper digestive tract, and the first and second cavities (102, 204) enclose respective drug substances for release in said environment.

8. A dosage form according to any of the claims 5 to 7 **characterized in that** the film means is hydroxyethyl cellulose or low-substituted hydroxypropyl cellulose.
